# EUROPEAN PATENT SPECIFICATION

(11) **EP 1 066 844 B1**
(45) Date of publication and mention of the grant of the patent: **05.10.2005**
(21) Application number: 00650074.8
(22) Date of filing: 30.06.2000
(51) Int. Cl.: A61M 5/14, A61M 5/44, H05B 3/58, F16L 53/00

(54) **Intravenous fluid heating system for heating the fluid in the infusion tube**
Intravenöse Flüssigkeitsheizvorrichtung zum Erwärmen der Flüssigkeit im Infusionsschlauch
Dispositif de chauffage du fluide de perfusion pour intraveineuse par chauffage du fluide dans le tube de perfusion

(30) Priority: 06.07.1999 US 347778
(43) Date of publication of application: 10.01.2001
(73) Proprietor: Smiths Medical ASD, Inc., Keene, NH 03431 (US)
(72) Inventor: Shigezawa, Gordon, Irvine, California 92714 (US)
(74) Representative: McCarthy, Denis Alexis

(56) References cited:
- EP-A- 0 296 777
- WO-A-96/11027
- DE-A- 19 716 977
- DE-A- 19 803 378
- US-A- 4 038 519
- US-A- 4 532 414
- US-A- 5 713 864
- US-E- R E35 501

## Description

### BACKGROUND OF THE INVENTION

### 1. Field of the Invention

The present invention relates generally to medical equipment, and more particularly to an intravenous fluid heating system including heat delivery along a length of tubing prior to introduction of the fluid into the patient to maintain and control an elevated fluid temperature.

### 2. Description of Related Art

Normothermia for humans is 37° C (98.6° F). When the body temperature falls below 36°, clinicians refer to the condition as hypothermic. Except for those rare procedures where hypothermia is a planned and carefully controlled surgical tactic for protecting the patient (e.g., open heart surgery and some neurosurgery), hypothermia is regarded as generally a disfavored, uncontrolled, and unintentional byproduct of medical procedures. However, the occurrence of hypothermia in post surgical recovery rooms can be as high as 60% to 70%. The outward manifestations of hypothermia can be shivering and discomfort, and the condition can lead to further complications.

There are many known contributing factors to post-surgical hypothermia. Cold operating rooms contribute to the patient's loss of heat. Most ORs are kept colder than normal rooms, typically maintaining a maximum temperature of 20° C. Another factor is the patient's lack of clothing during a surgical procedure. Many times a patient will be exposed to the cold operating room with at most a flimsy gown, and in some instances the patient is predominantly exposed during what can be a lengthy procedure. Evaporation of fluids applied to the body such as Iodine can further rob the patient's body of heat. Another significant loss of heat can be the heat exchange between a body which has been opened, exposing the vital organs, and the surrounding environment. These factors contribute to the high incidence of a patient's post-operative hypothermia.

An important contributor to the hypothermia problem is the introduction of intravenous (IV) fluids into the patient before, during, and after surgery. For example, blood products are stored in refrigerators at temperatures of 4° C prior to use, which is just above freezing. Other fluids such as saline or glucose solutions are stored at room temperature (20° C), which is approximately 17° below the body temperature. When a cold fluid is introduced into the body, the body must work to bring the new fluid to the body's operating temperature at the expense of other body functions. In doing so, the body cools below its initial temperature, with the amount of cooling dependent on the quantity and temperature of the fluid to be introduced. Large amounts of fluid or very cold fluids can cause the patient's temperature to drop several degrees, thereby triggering hypothermia even without any other contributing factors. This effect is magnified in younger patients as well as the elderly. Thus, the introduction of blood and other IV solutions are a major contributor to the problem of hypothermia in post-surgical patients.

In recognition of this problem, the medical community has tried to implement blood warmers which preheat the blood prior to introduction of the blood into the patient. However, blood warmers have heretofore been an unsatisfactory solution to the problem. First, while the existing blood warmers add some heat to the blood prior to delivery, the blood is still delivered at a temperature colder than the 37° - 38° C which is maintained by the human body. This is attributable to heat loss of the preheated blood in the line from the heater to the point of infusion, where the warm line gives off heat to the colder surrounding environment. If the flow rate of the fluid is slow, then more heat is lost during the exposure time between the heater and the infusion point.

The majority of prior art fluid warmers are limited by having the heated region separate from the point of infusion, where the heat source is separated by the venipuncture site by a length of IV tubing. The fluid cools down in the unheated line necessitating a higher initial temperature of the heated fluid. However, overheating the fluid can break down products in the fluid and in some cases render the fluid useless or unsafe. The cool-down is particularly severe at low flow rates where a long residency time in the post-heater connecting line results in heat energy loss that could be as much as the heat added. At higher flow rates, the heater response time of prior art heaters prevents rapid response to abrupt flow changes without overheating the fluid.

United States Patent Specification No. 5,713,864 discloses a flexible, integral polymer resistance-heated conduit that is particularly suitable for use with physiological fluids for uniformly heating the fluids and maintaining them at a predetermined temperature. A preferred embodiment disclosed in United States patent specification No. 5,713,864 includes a conduit that is made of at least two types of polymeric materials. The first material is transparent to allow the user to view the contents of the conduit for determining whether fluid is or is not flowing in the conduit. A second material is conductive and is heated by ohmic resistance when electric current is passed through it. The two materials are in intimate contact and are preferably coextruded to transfer heat from the conductive sections to the other sections.

### SUMMARY OF THE INVENTION

To offset the problem of heat loss in the tubing which transports the IV fluid from the heating unit to the patient, the present invention employs a tubing which comprises an internal heating web traversing the length of the tubing, which heats the fluid in the tubing and prevents the fluid from entering the patient below normothermia temperatures. In a preferred embodiment, the present invention includes proximal and distal sensors which evaluate the temperatures at the beginning of the tube and the point of delivery, thereby providing a feedback loop for controlling the temperature at the point of entry of the body. By placing a heating web inside the flow field of the moving fluid, the present invention advantageously heats the fluid more efficiently and more evenly than if the outer walls of the tubing were heated.

### BRIEF DESCRIPTION OF THE DRAWINGS

The exact nature of this invention, as well as its objects and advantages, will become readily apparent upon reference to the following detailed description when considered in conjunction with the accompanying drawings, in which like reference numerals designate like parts throughout the figures thereof, and wherein:
Figure 1 is a schematic illustration of a preferred embodiment of the present invention including a gravity assisted fluid flow, a heating unit, and a length of tubing with internal heating web;
Figure 2 is a second schematic illustration of a preferred embodiment of the present invention as previously depicted in Figure 1 with a pressure assisted fluid flow;
Figure 3 is a schematic illustration of a length of tubing with a heating web of the present invention, shown partially in phantom;
Figure 4 is a cross-sectional view of the tubing as depicted in Figure 3;
Figure 5 is an elevated, perspective view of the tubing, partially cut away, and the internal heating web of the present invention;
Figure 6 is a block diagram of a heater controller used in the heating unit of the present invention; and
Figure 7 is a block diagram of a second heater controller with dual microprocessors.

### DESCRIPTION OF THE PREFERRED EMBODIMENTS

The following description is provided to enable any person skilled in the art to make and use the invention and sets forth the best modes contemplated by the inventor of carrying out his invention. Various modifications, however, will remain readily apparent to those skilled in the art, since the general principles of the present invention have been defined herein specifically to provide an IV fluid heating system with internal flow heating using an internal web to transmit heat to the moving fluid.

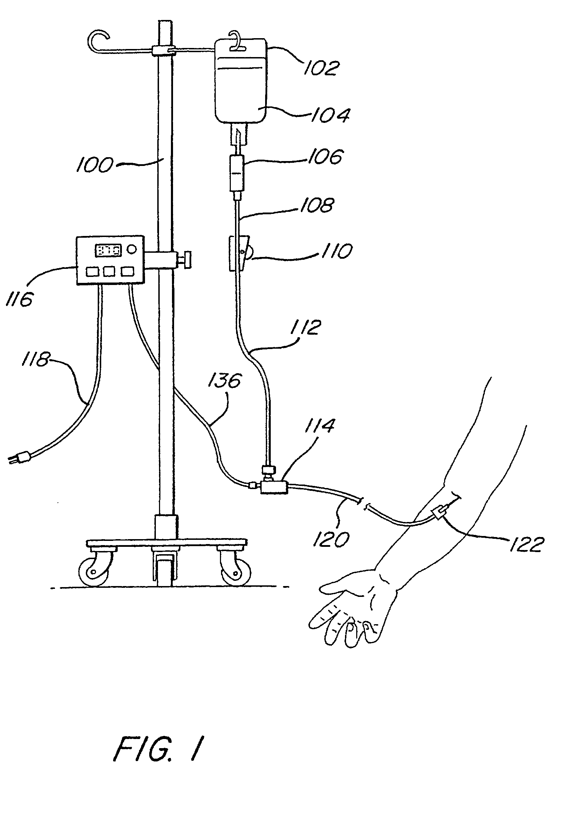

Figure 1 depicts a schematic of a fluid delivery system employing the present invention. An IV stand 100 supports a flexible container 102 which stores a fluid 104 such as blood or saline to be delivered to the patient. The fluid 104 feeds to a drip chamber 106 which accumulates the fluid before delivery to the patient. A tube 108 connecting the drip chamber 106 leads to a flow control valve 110 which regulates the flow rate of the fluid administered to the patient. A flexible tubing 112 connects the flow control valve 110 to a junction 114, which preferably includes a thermistor or other temperature sensor for detecting the initial temperature of the fluid. The junction 114 is connected electronically to a heat controlling unit 116 preferably mounted on the IV stand 100 as shown. The heat controlling unit 116 is powered by an AC current via its power line 118, or could alternatively be powered by a dc battery if necessary.

As fluid exits the junction 114, it travels along a tubing 120 to a cannula 122 or other means for introducing the IV fluid to the patient. Along this length of tubing 120, heat is continuously transferred to the fluid as will be explained in greater detail below. At or near the cannula 122, a second thermal sensor is provided which measures the temperature of the fluid immediately before the fluid's introduction into the patient, and this temperature is communicated back to the heat controlling unit 116. By adjusting the amount of heat which is introduced along the length of tubing 120, the fluid temperature can be accurately controlled and delivered at the proper temperature. In this manner, a contributor to hypothermia is diminished or eliminated.

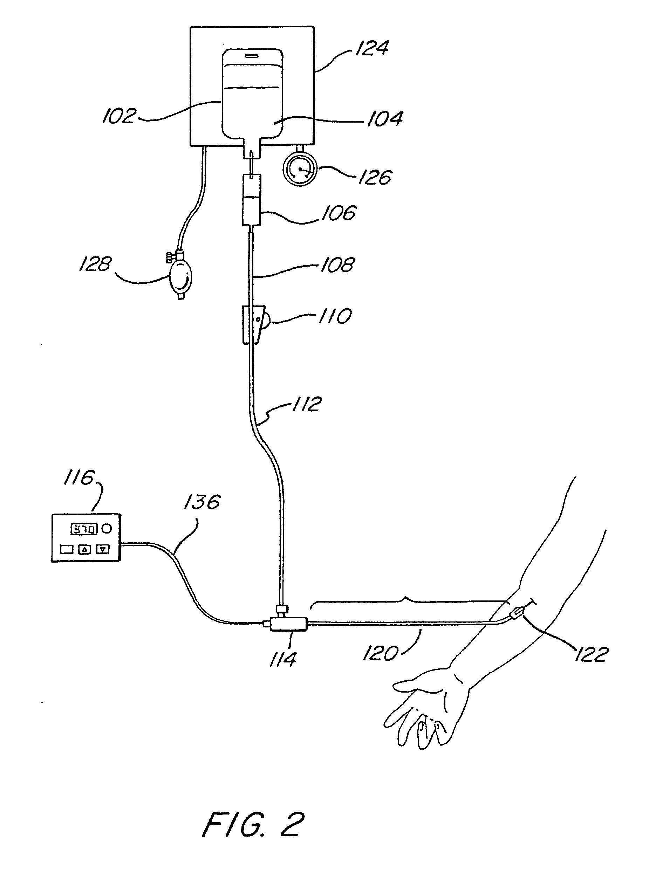

Figure 2 depicts a second embodiment to that shown in Figure 1, in which the intravenous fluid 104 is delivered using pressure in addition to gravity to control the flow rate. Using like numerals to represent like components, Figure 2 includes a pressure infuser 124 about the IV container 102 which imparts a pressure on the flexible container 102. A pressure gauge 126 attached to the pressure infuser 124 displays the pressure imparted on the container 102, which may be applied using either a manual delivery such as a hand pump 128, or a mechanical delivery such as a motor (not shown).

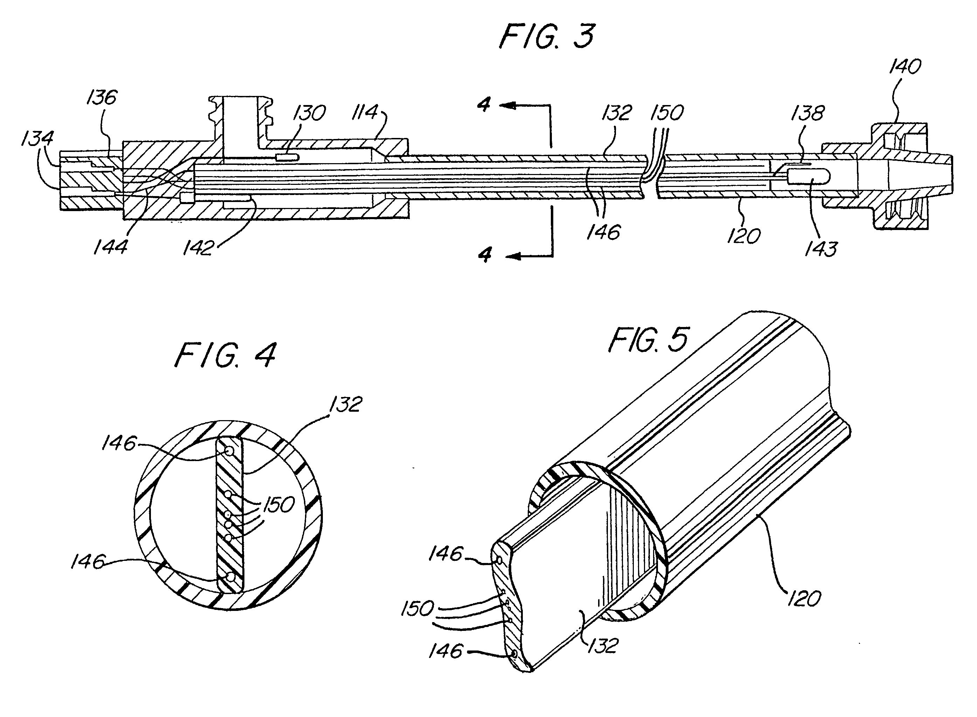

Figure 3 shows a cross-section of the tubing 120, including devices to measure the fluid temperature and apply heat to the moving fluid. The junction 114 receives the cold fluid from the flow rate controller and a thermal sensor 130 in the junction 114 measures the temperature of the cold fluid. This first thermal sensor 130 senses overtemperature in the fluid, and can also be used to determine the initial power setting for heating the fluid. A flexible web 132 extends from the tubing 120 into the junction 114, where electrical leads 134 are connected. The electrical leads 134 extend from an electrically insulating conduit 136 and extend to the heat controlling unit 116 as previously shown in Figure 1.

The tubing 120 further includes a second thermal sensor 138 at the proximal end (i.e., end nearest the patient), and the temperature measurements from the first and second thermal sensors are communicated back to the heat controlling unit 116 via the conduit 136. In a preferred embodiment, the proximal end includes a dual thermistor for redundancy, wherein a discrepancy between the two sensors forming the dual thermistor triggers an alarm that one of the thermistors has strayed from a predetermined tolerance. In this manner, a thermistor malfunction does not result in over-heated fluid being delivered to the patient. The tubing is preferably terminated at a "luer" connector 140, or similar attachment for facilitating the introduction of fluid into the patient.

Figure 3 also details first and second electrodes 142, 143 positioned at opposite ends of the web 132, and connected by an electrical conduit 144 embedded in the web 132. The electrodes 142, 143 detect discontinuities that may occur in the web 132, such as breaks, pinholes, insulation failure, and bubbles forming on the web. The formation of bubbles can give rise to excessive power dissipation and hot spots on the web, which may in turn damage the tubing 120.

Figures 4 and 5 show the web element 132 disposed in the tubing 120. The tubing itself may be of the type traditionally used for IVs, such as one-eighth inch polyvinyl chloride (PVC) tubing. The web 132 is flexible and preferably extruded along with the embedded heating elements 146, from a heat-resistant material, and spans the diameter of the tubing as shown. The tubing 120 and web 132 may be extruded together in a single configuration, or the web may be formed separate from the tubing and subsequently inserted therein. Where the web and tubing are formed together, the web diametrically spans the tubing, integrally forming intersections with the walls of the tubing at opposite sides of the tubing.

The web includes wires 146 to electrically heat the web 132, which in turn heats the fluid continuously along the tubing 120. Alternately, the wires 146 may traverse the web laterally as well as longitudinally in a zigzag pattern to provide more heat per length of tubing. By passing current through the wires 146, the dissipation of power will cause the wires 146 to heat up along the entire length of the wires and consequently the web 132 is heated. As the fluid continuously flows past the web 132, the heat from the web is transferred to the fluid via conduction and convection. The web 132 also carries wires 150 which convey signals from the thermistors 130, 138, preferably along the central portion of the web. The web must be thin enough to allow the tubing to flex, but sturdy enough to prevent the web from cracking or splitting along the length of tubing. A typical width of the web is on the order of one tenth of an inch, and is preferably made from a plastic such as pvc or an extrudable elastomer.

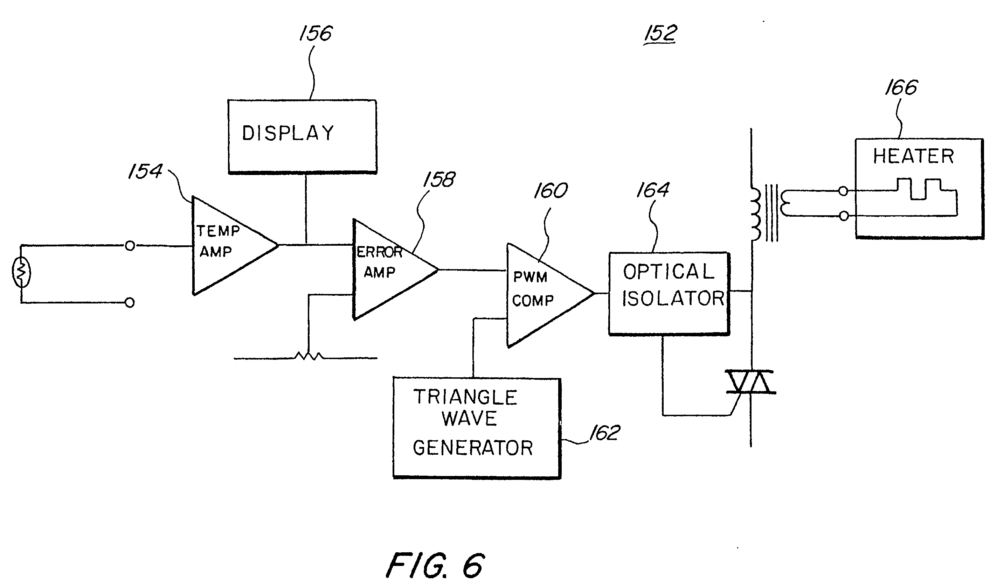

Figure 6 depicts a block diagram of a first embodiment feedback control circuit 152 employed by the present invention. The signal from the proximal thermistor is fed into an amplifier 154 which increases the signal strength, and the amplified signal is displayed at the display unit 156 . The temperature signal is directed to an error amplifier 158, which receives the optimum or desired temperature from the heating unit input and compares the two signals. The difference is then supplied to a pulse width modulator (pwm) comparator 160, along with a known signal such as a triangle wave generated from a triangle wave generator 162, and the output of the comparator 160 is fed to an optical isolator 164. A heater transformer 166 is controlled by the output of the optical isolator 164, which in turn controls the amount of current generated in the heating wires 146 in the web 132. As more heat is needed, the power to the transformer is adjusted to augment the electrical current, raising the power delivered to the wire and producing more heat. The increase in the heat is transferred to the fluid, which raises the downstream temperature at the proximal thermistor. In this manner, the optimum temperature at the proximal thermistor is maintained.

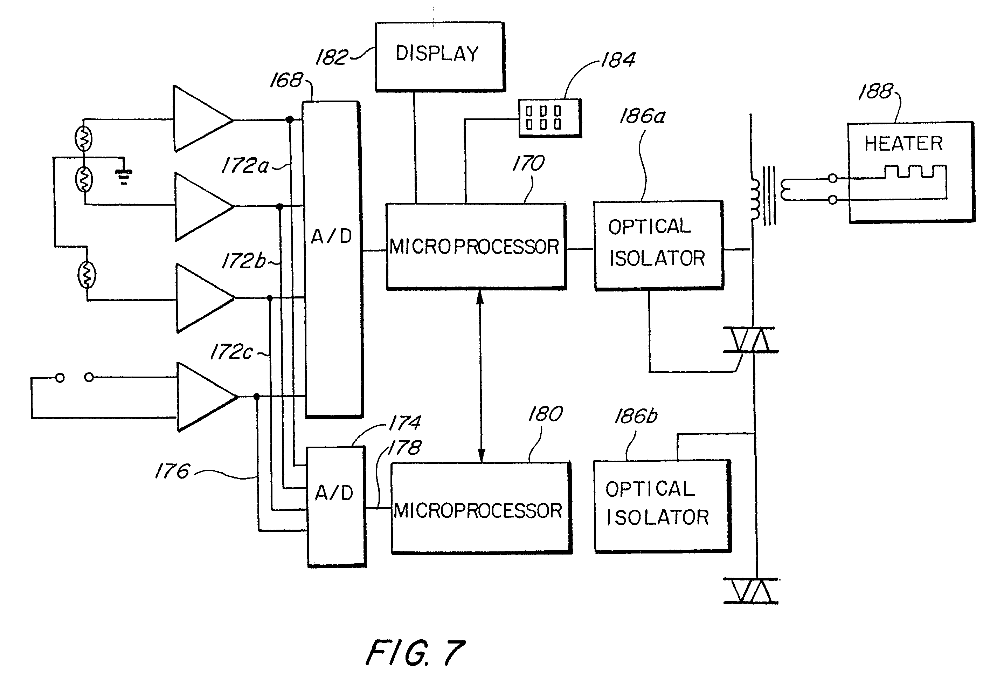

Figure 7 depicts a second feedback circuit comprising a dual microprocessor heat controller. In this circuit, signals from two proximal thermistors and one distal thermistor are converted to a digital signal via analog-to-digital converter 168 to a first microprocessor 170. In parallel, the three thermistor signals 172a, 172b, 172c are fed through a second analog-to-digital converter 174 along with a conductivity measurement 176, and the resultant digital signal 178 is directed to a second microprocessor 180 in communication with the first microprocessor 170. A display unit 182 and input device 184 are connected to the first microprocessor 170, which processes the digital signal and displays the temperatures at the display unit 182. The digital signals processed from the first and second microprocessors 170, 180 are each delivered to separate optical isolators 186a, 186b which test the signal using a primary triac and a secondary triac. The result of the test is used to adjust the power to the heater transformer 188, which in turn adjusts the current in the wires and the heat delivered to the fluid.

The disclosed heating system is a low mass, fast response time electrical heater that produces a flatter temperature versus flow characteristics profile as compared with fluid heaters using a heating jacket. Those skilled in the art will appreciate that various adaptations and modifications of the just-described preferred embodiment can be configured without departing from the scope of the invention. For example, the feedback control circuit could be modified from its described embodiments by those skilled in the art without departing from the scope of the invention. Similarly, other changes are possible while practicing the invention. Therefore, it is to be understood that, within the scope of the appended claims, the invention may be practiced other than as specifically described herein.

## Claims

1. A flexible medical tubing (120) for use in heating intravenous fluids and for conducting said intravenous fluid; comprising at least one heating element;
**characterized in that** said flexible medical tubing further comprises:
an internalflexible web (132) disposed in said flexible tubing to provide heat transfer surfaces in contact with the intravenous fluid, and **in that** said web (132) comprises said at least one heating element (146) embedded within said web (132), said at least one heating element (146) being spaced from said flexible tubing (120), and said web (132) traversing generally the length of flexible tubing (120) for communicating heat to the intravenous fluid.

2. The medical tubing of Claim 1 further comprising a first temperature sensor (138) disposed in said flexible tubing (120).

3. The medical tubing of Claim 2 wherein the first temperature sensor (138) is disposed at a proximal location to an end of the tubing (120) where the intravenous fluid is to be delivered.

4. The medical tubing of Claim 3 further comprising a redundant temperature sensor (138) disposed at the proximal location.

5. The medical tubing of Claim 3 further comprising a distal temperature sensor (130) disposed at a distal location to the end of the tubing where the intravenous fluid is to be delivered.

6. The medical tubing of Claim 1, wherein the internal web (132) comprises a first heat conducting wire (146) disposed along a first edge, and a second heat conducting wire (146) disposed along a second edge, and a third wire (150) communicating a temperature signal.

7. The medical tubing of Claim 1 further comprising a junction (114) disposed at an end of said length of tubing (120) distal to a location where said fluid is to be delivered, said junction (114) comprising a temperature sensor (130) for measuring the fluid entering the junction (114), and further comprising electrical connectors (134) cooperating with said internal web to provide an adjustable electric current to said internal web.

8. A system for delivering an intravenous fluid to a patient at an elevated, controlled temperature comprising a medical tubing. (120) as defined in any of Claims 1 to 7. the system further comprising:
a flexible container (102} for storing fluid, where the fluid is below a predetermined optimum delivery temperature;
a flow controller (110) for controlling the flow from the flexible container (102) to the flexible tubing (120);
a first tubing (112) connected to the flow controller (110);
a junction (114) connected to said first tubing (112), said junction (114) including an electrical connection and a conduit (136);
a heat controlling unit (116) for receiving temperature signals from the junction (114) and adjusting a current flow to the junction (114) in response to said signals, said conduit (136) connecting said junction (114) to said heat controlling unit (116); and
wherein the internal flexible web (132) of the medical tubing (120) is in electrical communication with said junction (114) wherein said current provided to the junction (114) by the heat controlling unit (116) is transferred to the web (132) which converts the current to heat and said heat is transferred to a fluid in said medical tubing (120).

9. The system of Claim 8 further comprising a first temperature sensor (138) located at a proximal end of said medical tubing (120) adjacent a delivery location, said first temperature sensor (138) providing a signal carried along said internal web (132) to said junction (114) and from there to said heat controlling unit (116) for use in adjusting the current flow.

10. The system of Claim 9 further comprising a second temperature sensor (130) located at a distal end of said medical tubing (120), said second temperature sensor (130) providing a signal to said heat controlling unit (116) for use in adjusting the current flow.

11. The system of Claim 8 wherein the flow of fluid is controlled solely by gravity,

12. The system of Claim 8 wherein the flow of fluid is controlled in part by a pumping apparatus (128).

## Patentansprüche

1. Ein flexibler medizinischer Schlauch (120) zur Verwendung beim Erwärmen intravenöser Fluide und zum Weiterleiten besagten intravenösen Fluids; welcher zumindest ein Heizelement umfasst;
**dadurch gekennzeichnet, dass** besagter flexibler medizinischer Schlauch weiterhin umfasst:
einen in besagtem flexiblen Schlauch angeordneten inneren flexiblen Steg (132) zur Verschaffung von Wärmeübertragungsoberflächen in Kontakt mit dem intravenösen Fluid, und **dadurch**, dass besagter Steg (132) besagtes zumindest eine Heizelement (146) in besagten Steg (132) eingebettet umfasst, wobei besagtes zumindest eine Heizelement (146) von besagtem flexiblen Schlauch (120) beabstandet ist und besagter Steg (132) im allgemeinen die Länge des flexiblen Schlauchs (120) durchläuft, um Wärme auf das intravenöse Fluid zu übertragen.

2. Der medizinische Schlauch gemäß Anspruch 1, der weiterhin einen in besagtem flexiblen Schlauch (120) angeordneten ersten Temperatursensor (138) umfasst.

3. Der medizinische Schlauch gemäß Anspruch 2, wobei der erste Temperatursensor (138) an einer proximalen Stelle zu einem Ende des Schlauchs (120), wo das intravenöse Fluid abgegeben werden muss, angeordnet ist.

4. Der medizinische Schlauch gemäß Anspruch 3, der weiterhin einen an der proximalen Stelle angeordneten überzähligen Temperatursensor (138) umfasst.

5. Der medizinische Schlauch gemäß Anspruch 3, der weiterhin einen an einer distalen Stelle zu dem Ende des Schlauchs, wo das intravenöse Fluid abgegeben werden muss, angeordneten distalen Temperatursensor (130) umfasst.

6. Der medizinische Schlauch gemäß Anspruch 1, wobei der innere Steg (132) einen entlang einer ersten Kante angeordneten ersten wärmeleitenden Draht (146) und einen entlang einer zweiten Kante angeordneten zweiten wärmeleitenden Draht (146) und einen ein Temperatursignal übertragenden dritten Draht (150) umfasst.

7. Der medizinische Schlauch gemäß Anspruch 1, der weiter ein an einem Ende besagten Schlauchstücks (120) distal zu einer Stelle, wo besagtes Fluid abgegeben werden muss, angeordnetes Verbindungsstück (114) umfasst, wobei besagtes Verbindungsstück (114) einen Temperatursensor (130) zum Messen des in das
weiter elektrische Anschlussstücke (134) umfasst, die mit besagtem inneren Steg zusammenwirken, um besagtem inneren Steg einen regelbaren elektrischen Strom zuzuführen.

8. Ein System zur Abgabe eines intravenösen Fluids an einen Patienten auf einer erhöhten, kontrollierten Temperatur, das einen medizinischen Schlauch (120) umfasst, wie in einem der Ansprüche 1 bis 7 definiert, wobei das System weiterhin folgendes umfasst:
einen flexiblen Behälter (102) zur Lagerung von Fluid, wobei das Fluid unter einer vorbestimmten optimalen Abgabetemperatur ist;
einen Durchflussregler (110) zur Steuerung des Flusses aus dem flexiblen Behälter (102) zu dem flexiblen Schlauch (120);
einen an den Durchflussregler (110) angeschlossenen ersten Schlauch (112);
ein an besagten ersten Schlauch (112) angeschlossenes Verbindungsstück (114), wobei besagtes Verbindungsstück (114) einen elektrischen Anschluss und eine Leitung (136) enthält;
eine Wärmeregeleinheit (116) zum Empfangen von Temperatursignalen von dem Verbindungsstück (114) und Regulieren eines Stromflusses zu dem Verbindungsstück (114) in Reaktion auf besagte Signale, wobei besagte Leitung (136) besagtes Verbindungsstück (114) mit besagter Wärmeregeleinheit (116) verbindet; und
wobei der innere flexible Steg (132) des medizinischen Schlauchs (120) in elektrischer Verbindung mit besagtem Verbindungsstück (114) steht, wobei besagter, dem Verbindungsstück (114) von der Wärmeregeleinheit (116) zugeführte Strom auf den Steg (132) übertragen wird, der den Strom in Wärme umwandelt, und besagte Wärme auf ein Fluid in besagtem medizinischen Schlauch (120) übertragen wird.

9. Das System gemäß Anspruch 8, das weiterhin einen an einem proximalen Ende besagten medizinischen Schlauchs (120) benachbart zu einer Abgabestelle befindlichen ersten Temperatursensor (138) umfasst, wobei besagter Temperatursensor (138) ein Signal bereitstellt, das entlang besagtem inneren Steg (132) zu besagtem Verbindungsstück (114) und von dort zu besagter Wärmeregeleinheit (116) zur Verwendung beim Regulieren des Stromflusses übertragen wird.

10. Das System gemäß Anspruch 9, das weiterhin einen an einem distalen Ende besagten medizinischen Schlauchs (120) befindlichen zweiten Temperatursensor (130) umfasst, wobei besagter zweiter Temperatursensor (130) ein Signal zu besagter Wärmeregeleinheit (116) zur Verwendung beim Regulieren des Stromflusses bereitstellt.

11. Das System gemäß Anspruch 8, wobei der Fluidstrom ausschließlich durch die Gravitation gesteuert wird.

12. Das System gemäß Anspruch 8, wobei der Fluidstrom teilweise durch eine Pumpvorrichtung (128) gesteuert wird.

## Revendications

1. Tubulure médicale flexible (120) à utiliser dans le chauffage de fluides intraveineux et pour la conduction dudit fluide intraveineux, comprenant au moins un élément de chauffage,
**caractérisée en ce que** ladite tubulure médicale flexible comprend en outre :
une paroi flexible interne (132) disposée dans ladite tubulure flexible pour procurer des surfaces de transfert thermique en contact avec le fluide intraveineux, et **en ce que** ladite paroi (132) comprend ledit au moins un élément de chauffage (146) à l'état encastré dans ladite paroi (132), ledit au moins un élément de chauffage (146) étant espacé par rapport à ladite tubulure flexible (120), et ladite paroi (132) s'étendant en général sur toute la longueur de la tubulure flexible (120) afin de communiquer de la chaleur au fluide intraveineux.

2. Tubulure médicale selon la revendication 1, comprenant en outre un premier capteur de température (138) disposé dans ladite tubulure flexible (120).

3. Tubulure médicale selon la revendication 2, dans laquelle le premier capteur de température (138) est disposé à un endroit proximal par rapport à l'extrémité de la tubulure (120) à laquelle le fluide intraveineux doit être distribué.

4. Tubulure médicale selon la revendication 3, comprenant en outre un capteur de température redondant (138) disposé à l'endroit proximal.

5. Tubulure médicale selon la revendication 3, comprenant en outre un capteur de température distal (130) disposé à un endroit distal par rapport à l'extrémité de la tubulure à laquelle le fluide intraveineux doit être distribué.

6. Tubulure médicale selon la revendication 1, dans laquelle la paroi interne (132) comprend un premier fil métallique thermoconducteur (146) disposé le long d'un premier bord, un deuxième fil métallique thermoconducteur (146) disposé le long d'un deuxième bord et un troisième fil métallique (150) communiquant un signal de température.

7. Tubulure médicale selon la revendication 1, comprenant en outre un raccord (114) disposé à une extrémité de ladite longueur de tubulure (120) en position distale par rapport à l'endroit auquel ledit fluide doit être distribué, ledit raccord (114) comprenant un capteur de température (130) pour mesurer le fluide pénétrant dans le raccord (114), et comprenant en outre des connecteurs électriques (134) coopérant avec ladite paroi interne pour procurer un courant électrique réglable à ladite paroi interne.

8. Système pour distribuer un fluide intraveineux à un patient à une température élevée contrôlée comprenant une tubulure médicale (120) telle que définie dans l'une quelconque des revendications 1 à 7, le système comprenant en outre :
un récipient flexible (102) pour stocker du fluide, la température du fluide étant inférieure à une température de distribution optimale prédéterminée ;
un régulateur du débit (110) pour régler le débit entre le récipient flexible (102) et la tubulure flexible (120) ;
une première tubulure (112) connectée au régulateur du débit (110) ;
un raccord (114) connecté à ladite première tubulure (112), ledit raccord (114) englobant une connexion électrique et un conduit (136) ;
une unité de réglage thermique (116) pour la réception de signaux de température émis par le raccord (114) et pour régler une circulation de courant en direction du raccord (114) en réponse auxdits signaux, ledit conduit (136) reliant ledit raccord (114) à ladite unité de réglage thermique (116) ; et
dans lequel la paroi flexible interne (132) de la tubulure médicale (120) est mise en communication électrique avec ledit raccord (114), ledit courant fourni au raccord (114) par l'unité de réglage thermique (116) étant transféré à la paroi (132) qui transforme le courant en chaleur et ladite chaleur étant transférée à un fluide dans ladite tubulure médicale (120).

9. Système selon un revendication 8, comprenant un premier capteur de température (138) disposé à l'extrémité proximale de ladite tubulure médicale (120) en position adjacente à un endroit de distribution, ledit premier capteur de température (138) fournissant un signal transporté le long de ladite paroi interne (132) audit raccord (114) et, de là, à ladite unité de réglage thermique (116) à utiliser pour régler la circulation du courant.

10. Système selon un revendication 9, comprenant en outre un deuxième capteur de température (130) disposé à l'extrémité distale de ladite tubulure médicale (120), ledit deuxième capteur de température (130) fournissant un signal à ladite unité de réglage thermique (116) à utiliser pour régler la circulation du courant.

11. Système selon la revendication 8, dans lequel l'écoulement de fluide est réglé uniquement par la force de gravité.

12. Système selon la revendication 8, dans lequel l'écoulement de fluide est réglé en partie par un appareil de pompage (128).
